# EUROPÄISCHE PATENTSCHRIFT

(11) **EP 0 759 430 B1**
(45) Veröffentlichungstag und Bekanntmachung des Hinweises auf die Patenterteilung: **06.05.1999**
(21) Anmeldenummer: 96112663.8
(22) Anmeldetag: 06.08.1996
(51) Int. Cl.: C07D 249/12

(54) **Verfahren zur Herstellung von substituierten Aminotriazolinonen**
Process for preparing substituted aminotriazolinones
Procédé pour la préparation d'aminotriazolinones

(30) Priorität: 18.08.1995 DE 19530450
(43) Veröffentlichungstag der Anmeldung: 26.02.1997
(73) Patentinhaber: BAYER AG, 51368 Leverkusen (DE)
(72) Erfinder: Diehr, Hans-Joachim, Dr., 42329 Wuppertal (DE); Müller, Klaus-Helmut, Dr., 40593 Düsseldorf (DE); Lantzsch, Reinhard, Dr., 42115 Wuppertal (DE)

(56) Entgegenhaltungen:
- EP-A- 0 301 946
- EP-A- 0 321 833
- EP-A- 0 422 469

## Beschreibung

Die Erfindung betrifft ein neues Verfahren Zur Herstellung von substituierten Aminotriazolinonen, welche als Ausgangsstoffe für herbizid wirksame Verbindungen bekannt sind.

Es ist bekannt, daß man bestimmte substituierte Aminotriazolinone, wie z.B. die Verbindung 4-Amino-5-methyl-2,4-dihydro-3H-1,2,4-triazol-3-on erhält, wenn man entsprechende Oxadiazolone, wie z.B. die Verbindung 5-Methyl-1,3,4-oxadiazol-2(3H)-on mit Hydrazinhydrat in Wasser umsetzt. (vgl. EP 321833).

Bei dieser Herstellungsweise wird das Oxadiazolon in Wasser vorgelegt und das Hydrazinhydrat (in hohem Überschuß) bei Raumtemperatur dazu gegeben und dann längere Zeit zum Sieden erhitzt. Überschüssiges Hydrazin muß durch Abdestillieren entfernt werden und man erhält die gewünschten Aminotriazolinone in nicht befriedigenden Ausbeuten und ungenügender Reinheit, so daß sich ein weiterer Reinigungsschritt, z.B. Azomethinbildung anschließen muß.

Es wurde nun gefunden, daß man substituierte Aminotriazolinone der allgemeinen Formel (I) 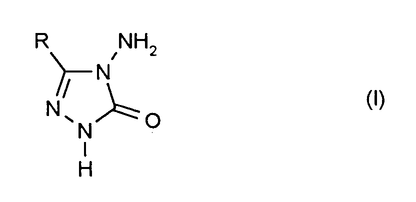 in welcher
- R: für einen jeweils gegebenenfalls durch Cyano, Halogen oder C₁-C₄ Alkoxy substituierten Rest der Reihe Alkyl, Alkoxy, Alkylthio, Alkylamino oder Dialkylamino mit jeweils bis zu 6 Kohlenstoffatomen steht,
in guten Ausbeuten und in hoher Reinheit erhält, wenn man
Hydrazinhydrat in Gegenwart einer (gegebenenfalls in Wasser gelösten) basischen Verbindung und in Gegenwart eines polaren organischen Lösungsmittels bei Temperaturen zwischen 90°C und 150°C vorlegt, innerhalb dieses Temperaturbereichs ein Oxadiazolon der allgemeinen Formel (II) 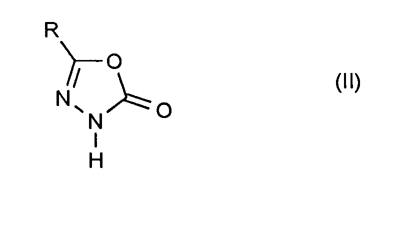 in welcher
- R: die oben angegebene Bedeutung hat,
langsam zudosiert und die Reaktionsmischung im oben angegebenen Temperaturbereich hält, bis die Umsetzung praktisch abgeschlossen ist.

Überraschenderweise können die substituierten Aminotriazolinone der allgemeinen Formel (I) nach dem erfindungsgemäßen Verfahren, bei welchem das Hydrazinhydrat in etwa äquimolarer Menge eingesetzt werden kann, in erheblich höheren Ausbeuten und in wesentlich besserer Qualität als nach dem bekannten Stand der Technik hergestellt werden.

Das erfindungsgemäße Verfahren stellt somit eine wertvolle Bereicherung des Standes der Technik dar.

Das erfindungsgemäße Verfahren betrifft insbesondere die Herstellung von Verbindungen der Formel (I), in welcher
- R: für einen jeweils gegebenenfalls durch Cyano, Fluor, Chlor, Brom, Methoxy oder Ethoxy substituierten Rest der Reihe Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n-oder i-Propylamino, n-, i-, s- oder t-Butylamino, Dimethylamino, Diethylamino oder Dipropylamino steht.

Verwendet man beispielsweise 5-Methyl-1,3,4-oxadiazol-2(3H)-on und Hydrazinhydrat als Ausgangsstoffe, so kann der Reaktionsablauf beim erfindungsgemäßen Verfahren durch das folgende Formelschema skizziert werden:

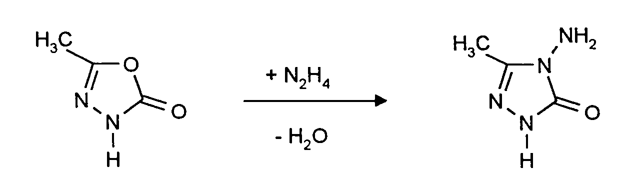

Die beim erfindungsgemäßen Verfahren zur Herstellung der Verbindungen der Formel (I) als Ausgangsstoffe zu verwendenden Oxadiazolone sind durch die Formel (II) allgemein definiert. In der Formel (II) hat R vorzugsweise bzw. insbesondere diejenige Bedeutung, die bereits oben bei der Beschreibung der erfindungsgemäß herzustellenden Verbindungen der Formel (I) als bevorzugt bzw. als insbesondere bevorzugt für R angegeben wurde.

Die Oxadiazolone der Formel (II) sind bekannt und/oder können nach an sich bekannten Verfahren hergestellt werden (vgl. EP 321833, Justus Liebigs Ann. Chem. 1973, 1816-1820).

Das erfindungsgemäße Verfahren wird in Gegenwart einer (gegebenenfalls in Wasser gelösten) basischen Verbindung durchgeführt. Als basische Verbindungen kommen in diesem Zusammenhang im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kaliummethanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8-Diazabicyclo[5,4,0]-undec-7-en (DBU).

Alkalimetallhydroxide wie Lithiumhydroxid, Natriumhydroxid und Kaliumhydroxid (insbesondere Natriumhydroxid - gegebenenfalls in Wasser gelöst) werden als basische Verbindungen bei der Durchführung des erfindungsgemäßen Verfahren besonders bevorzugt eingesetzt.

Das erfindungsgemäße Verfahren wird in Gegenwart eines polaren organischen Lösungsmittels durchgeführt. Hierzu gehören vorzugsweise Dialkylether, wie beispielsweise Diisopropylether, Methyl-t-butylether (MTBE), Ethyl-t-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -i-propylester, -n-, -i- oder - s-butylester; Alkohole, wie beispielsweise Ethanol, n- oder i-Propanol, n-, i-, s- oder t-Butanol, sowie Sulfoxide, wie beispielsweise Dimethylsulfoxid.

Alkohole, wie Methanol, Ethanol, n- oder i-Propanol, n-, i-, s- oder t-Butanol (insbesondere n-Butanol) werden als Lösungsmittel beim erfindungsgemäßen Verfahren besonders bevorzugt eingesetzt.

Die Reaktionstemperaturen können bei der Durchführung des erfindungsgemäßen Verfahrens in einem größeren Bereich variiert werden, insbesondere zwischen 90°C und 150°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen. Bei Verwendung niedrig siedender Alkohole wird vorzugsweise unter erhöhtem Druck gearbeitet.

Zur Durchführung des erfindungsgemäßen Verfahrens setzt man je Mol Oxadiazolon der Formel (II) zwischen 0,8 und 1,5 Mol, vorzugsweise zwischen 0,9 und 1,2 Mol, insbesondere zwischen 1,0 und 1,1 Mol Hydrazinhydrat und zwischen 0,01 und 1 Mol, vorzugsweise zwischen 0,05 und 0,50 Mol, insbesondere zwischen 0,10 und 0,25 Mol an basischer Verbindung ein.

Das Hydrazinhydrat wird im allgemeinen bei Raumtemperatur mit der (gegebenenfalls in Wasser gelösten) basischen Verbindung und dem polaren organischen Lösungsmittel vermischt und die Mischung wird auf die erforderliche Reaktionstemperatur aufgeheizt. Dann wird das Oxadiazolon der Formel (II) langsam zudosiert und die Reaktionsmischung wird - gegebenenfalls unter Rühren - weiter im angegebenen Temperaturbereich gehalten, bis die Umsetzung praktisch abgeschlossen ist.

Die Aufarbeitung und Isolierung der Produkte der Formel (I) ist problematisch, da sie sich in Wasser sehr gut, in organischen Lösungsmitteln jedoch sehr schlecht lösen. Wenn man beispielsweise - gegebenenfalls nach Absenken der Temperatur - mit Wasser verdünnt und durch Zugabe einer Säure wie beispielsweise Salzsäure oder Schwefelsäure auf pH 7 einstellt, dann das Lösungsmittel und einen Teil des Wassers - gegebenenfalls unter vermindertem Druck - weitgehend abdestilliert, bleibt das gewünschte Produkt als Rückstand und kann durch Abfiltrieren isoliert werden, wobei allerdings auf Grund der guten Wasserlöslichkeit ein Teil des Produktes im Wasser verbleibt und verloren geht.

In einer bevorzugten Ausführungsform der Aufarbeitung wird daher der pH-Wert durch Zugabe einer Säure auf 7 gestellt und dann entweder das beim Verfahren verwendete Lösungsmittel, das vorzugsweise nicht oder nur wenig mit Wasser mischbar ist, direkt oder mit einem anderen geeigneten Lösungsmittel bei erhöhter Temperatur kontinuierlich extrahiert. Die Temperatur liegt zwischen 30° und 100°C, vorzugsweise zwischen 45° und 90°C.

Neben den vorzugsweise verwendeten Alkoholen wie n- oder iso-Propanol, n-, iso-, sek.- oder tert.-Butanol können vorzugsweise Ester, wie beispielsweise Essigsäuremethyl-oder -ethylester zur kontinuierlichen Extraktion verwendet werden.

Überraschenderweise erhält man das gewünschte Produkt quantitativ und in hoher Reinheit. In der Wasserphase ist kein Produkt der Formel (I) mehr enthalten.

Die nach dem erfindungsgemäßen Verfahren herzustellenden substituierten Aminotriazolinone der allgemeinen Formel (I) sind bereits als Ausgangsstoffe für herbizid wirksame Verbindungen bekannt (vgl. EP 294 666, EP 370 293).

### Herstellungsbeispiele:

### Beispiel 1

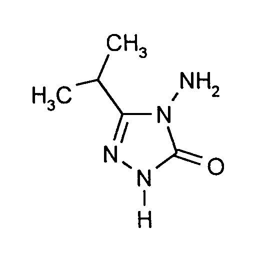

118,4 g (0,914 Mol) 5-i-Propyl-1,3,4-oxadiazol-2(3H)-on werden innerhalb von ca. 25 Minuten zu einer auf 102°C bis 105°C aufgeheizten Mischung aus 45,8 g (0,915 Mol) Hydrazinhydrat, 12,2 g 45%iger Natronlauge (0,137 Mol NaOH) und 460 ml n-Butanol tropfenweise unter Rühren gegeben und die Reaktionsmischung wird dann 3 Stunden im Temperaturbereich zwischen 100°C und 105°C gehalten. Nach Abkühlen auf ca. 50°C wird mit 230 ml Wasser verdünnt und dann bei ca. 20°C durch Zugabe von ca. 45%iger Schwefelsäure der pH-Wert auf 7 eingestellt. Das Lösungsmittel wird anschließend, zunächst bei Normaldruck, dann bei einem Druck zwischen 100 mbar und 110 mbar (Badtemperatur: ca. 60°C), weitgehend abdestilliert, der verbleibende Rückstand in 230 ml Wasser bei ca. 75°C gelöst, und die Lösung auf 10°C bis 20°C abgekühlt. Das hierbei kristallin angefallene Produkt wird durch Abfiltrieren isoliert, mit 20 ml Eiswasser gewaschen und im Wasserstrahlvakuum bei 60°C getrocknet.

Man erhält 105 g (Gehalt: 98%, d.h. Ausbeute: 79% der Theorie) 4-Amino-5-i-propyl-2,4-dihydro-3H-1,2,4-triazol-3-on vom Schmelzpunkt 171°C.

### Beispiel 2

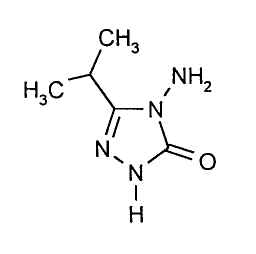

118,4 g (0,914 Mol) 5-i-Propyl-1,3,4-oxadiazol-2(3H)-on werden innerhalb von ca. 25 Minuten zu einer auf 102°C bis 105°C aufgeheizten Mischung aus 45,8 g (0,915 Mol) Hydrazinhydrat, 12,2 g 45%iger Natronlauge (0,137 Mol NaOH) und 460 ml n-Butanol tropfenweise unter Rühren gegeben und die Reaktionsmischung wird dann 3 Stunden im Temperaturbereich zwischen 100°C und 105°C gehalten. Nach Abkühlen auf ca. 20°C wird mit 100 ml Wasser verdünnt und mit 33%iger Salzsäure auf pH 7 gestellt. Nach Zugabe von weiteren 200 ml Wasser erhält man ein Klares Zweiphasengemisch aus Butanol und Wasser. Durch kontinuierliche Extraktion der Wasserphase mit dem Butanol bei 80°C kann man das gesamte Reaktionsprodukt in der organischen Phase ansammeln, während die bei der Reaktion entstehenden Nebenprodukte im Wasser verbleiben.

Durch Abdestillieren des Butanols erhält man 118 g (Gehalt: 98,3%, d.h. Ausbeute 89,3% der Theorie) 4-Amino-5-i-propyl-2,4-dihydro-3H-1,2,4-triazol-3-on vom Schmelzpunkt 171°C.

## Patentansprüche

1. Verfahren zur Herstellung von substituierten Aminotriazolinonen der allgemeinen Formel (I) 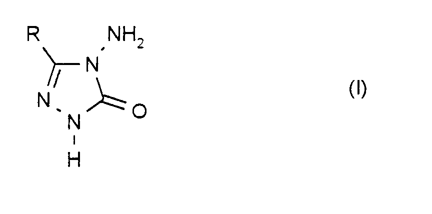 in welcher
R für einen jeweils gegebenenfalls durch Cyano, Halogen oder C₁-C₄-Alkoxy substituierten Rest der Reihe Alkyl, Alkoxy, Alkylthio, Alkylamino oder Dialkylamino mit jeweils bis zu 6 Kohlenstoffatomen steht,
dadurch gekennzeichnet, daß man
je Mol Oxadiazolon der allgemeinen Formel (II) zwischen 0,8 und 1,5 Mol Hydrazinhydrat und zwischen 0,01 und 1 Mol einer (gegebenenfalls in Wasser gelösten) basischen Verbindung in Gegenwart eines polaren organischen Losungsmittels bei Temperaturen zwischen 90°C und 150°C vorlegt, innerhalb dieses Temperaturbereichs ein Oxadiazolon der allgemeinen Formel (II) 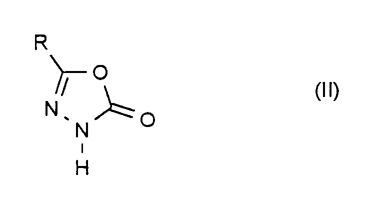 in welcher
R die oben angegebene Bedeutung hat,
langsam zudosiert und die Reaktionsmischung im oben angegebenen Temperaturbereich hält, bis die Umsetzung praktisch abgeschlossen ist.

2. Verfahren gemäß Anspruch 1, dadurch gekennzeichnet, daß
R für einen jeweils gegebenenfalls durch Cyano, Fluor, Chlor, Brom, Methoxy oder Ethoxy substituierten Rest der Reihe Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino, Diethylamino oder Dipropylamino steht.

3. Verfahren gemäß Anspruch 1, dadurch gekennzeichnet, daß man das Verfahren unter Normaldruck durchführt.

## Claims

1. Process for the preparation of substituted aminotriazolinones of the general formula (I) 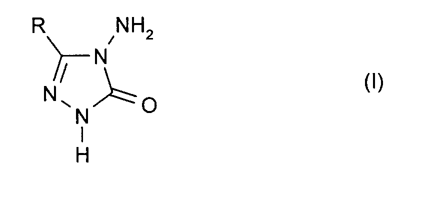 in which
R represents a radical of the series alkyl, alkoxy, alkylthio, alkylamino or dialkylamino each having up to 6 carbon atoms, each of which is optionally substituted by cyano, halogen or C₁-C₄-alkoxy,
characterized in that
per mole of oxadiazolone of the general formula (II) between 0.8 and 1.5 mol of hydrazine hydrate and between 0.01 and 1 mol of a basic compound (optionally dissolved in water) are initially introduced in the presence of a polar organic solvent at temperatures between 90°C and 150°C, an oxadiazolone of the general formula (II) 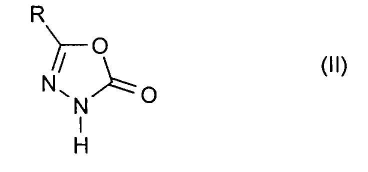 in which
R has the meaning indicated above,
is slowly metered in within this temperature range and the reaction mixture is kept in the temperature range indicated above until the reaction is virtually complete.

2. Process according to Claim 1, characterized in that
R represents a radical of the series methyl, ethyl, n- or i-propyl, n-, i-, s-or t-butyl, n-, i-, s- or t-pentyl, methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, dimethylamino, diethylamino or dipropylamino, each of which is optionally substituted by cyano, fluorine, chlorine, bromine, methoxy or ethoxy.

3. Process according to Claim 1, characterized in that the process is carried out under normal pressure.

## Revendications

1. Procédé pour la préparation d'aminotriazolinones substituées répondant à la formule générale (I) 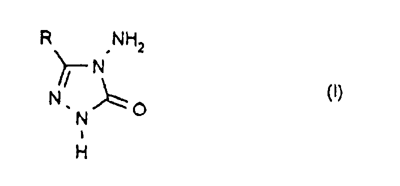 dans laquelle
R représente un radical choisi parmi la série comprenant un radical alkyle, un radical alcoxy, un radical alkylthio, un radical alkylamino ou un radical dialkylamino, chacun de ces radicaux contenant jusqu'à 6 atomes de carbone, et portant le cas échéant un ou plusieurs substituants identiques ou différents cyano, halogéno ou alcoxy on C₁-C₄,
caractérisé en ce qu'on dépose au préalable, par moles d'oxadiazolone répondant à la formule générale (II), entre 0,8 et 1,5 mole d'hydrate d'hydrazine et entre 0,01 et 1 mole d'un composé basique (le cas échéant, dissous dans l'eau) en présence d'un solvant organique polaire à des températures entre 90°C et 150°C; au sein de ce domaine de température, on ajoute lentement de manière dosée une oxadiazolone répondant à la formule générale (II) 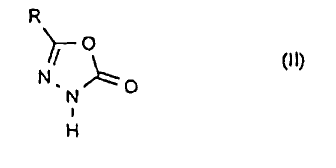 dans laquelle
R a la signification indiquée ci-dessus,
et on maintient le mélange réactionnel dans le domaine de température indiqué ci-dessus jusqu'à ce que la mise en réaction arrive pratiquement à son terme.

2. Procédé selon la revendication 1, caractérisé en ce que
R représente un radical choisi parmi la série comprenant un radical méthyle, un radical éthyle, un radical n- ou i-propyle, un radical n-, i-, s- ou t-butyle, un radical n-, i-, s-ou t-pentyle, un radical méthoxy, un radical éthoxy, un radical n- ou i-propoxy, un radical n-, i-, s- ou t-butoxy, un radical méthylthio, un radical éthylthio, un radical n- ou i-propylthio, un radical n-, i-, s- ou t-butylthio, un radical méthylamino, un radical éthylamino, un radical n- ou i-propylamino, un radical n-, i-, s- ou t-butylamino, un radical diméthylamino, un radical diéthylamino ou un radical dipropylamino, chacun de ces radicaux portant le cas échéant un ou plusieurs substituants identiques ou différents cyano, fluoro, chloro, bromo, méthoxy ou éthoxy.

3. Procédé selon la revendication 1, caractérisé en ce qu'on effectue le procédé sous pression normale.
